# EUROPEAN PATENT APPLICATION

(11) **EP 3 323 802 A1**
(43) Date of publication of application: **23.05.2018**
(21) Application number: 16199439.7
(22) Date of filing: 18.11.2016
(51) Int. Cl.: C07C 45/51

(54) **NOVEL USE OF ARYLPHOSPHORIC ACID DERIVATIVES AS CATALYST**

(71) Applicant: DSM IP Assets B.V., 6411 TE Heerlen (NL)
(72) Inventor: AQUINO, Fabrice, 4303 Kaiseraugst (CH); BONRATH, Werner, 4303 Kaiseraugst (CH); RIEBEL, Peter Hans, 4303 Kaiseraugst (CH)
(74) Representative: Dux, Roland

(57) **Abstract**

The present invention is directed towards a process for the manufacture of gamma,delta-unsaturated ketones of the general formula (III) 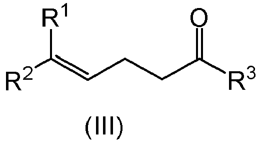 by reacting a tertiary vinyl carbinol of the general formula (I) with an isopropenyl alkyl ether of the general formula (II) in the presence of a catalyst of the general formula (IV), 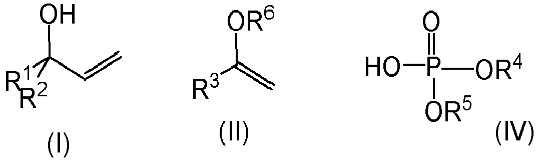 wherein R¹ and R⁶ are independently from each other either methyl or ethyl, R³ is methyl,
R² is selected from the group consisting of methyl, ethyl, homoprenyl, homogeranyl, homoneryl, homofarnesyl, and homosolanesyl, and R⁴ and R⁵ are independently from each other selected from the group consisting of hydrogen and aryl, or R⁴ and R⁵ form together 1,1'-binaphthyl-2,2'-diyl or mono-, di-, tri- or tetra-substituted 1,1'-binaphthyl-2,2'-diyl of formula V, whereby R^{A} and R^{A'} are independently from each other selected from the group consisting of methyl, phenyl, 4-nitrophenyl, 4-trifluoromethylphenyl, 3,5-bis(trifluoromethyl)phenyl, 9-anthracenyl and 2,4,6-trimethylphenyl, and R^{B} and R^{B'} are independently from each other selected from the group consisting of bromo, iodo, nitro, phenyl, methoxy and 3,5-bis(trifluoromethyl)phenyl, 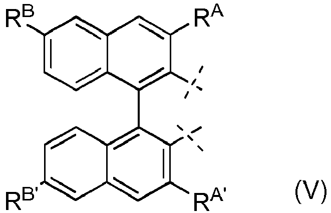 with the proviso that at least one of R⁴ and R⁵ is not hydrogen.

The present invention is also directed towards the reaction mixture as such, i.e. the mixture of the compound of formula (I), the compound of formula (II) and the catalyst of formula (IV).

## Description

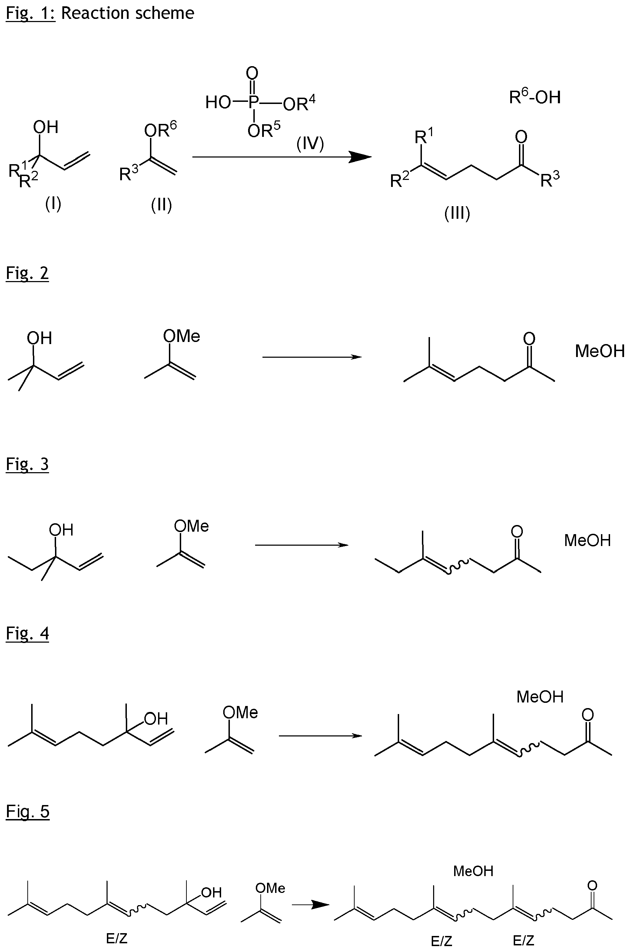

The present invention is directed towards an industrial sustainable process for the manufacture of gamma,delta-unsaturated ketones of formula (III) in the presence of novel catalysts of formula (IV), 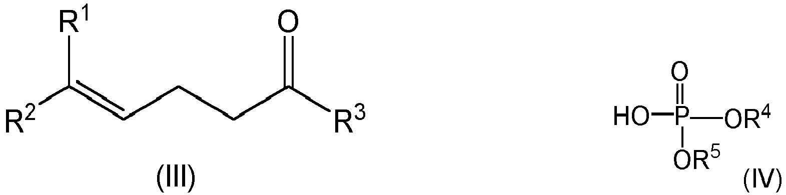 wherein R¹ is methyl or ethyl, R³ is methyl,
R² is selected from the group consisting of methyl, ethyl, homoprenyl (= "-CH₂-prenyl"), homogeranyl (="-CH₂-geranyl"), homoneryl (="-CH₂-neryl"), homofarnesyl (= "-CH₂-farnesyl"), and homosolanesyl ("-CH₂-solanesyl"),
R⁴ and R⁵ are independently from each other selected from the group consisting of hydrogen and aryl, or R⁴ and R⁵ form together 1,1'-binaphthyl-2,2'-diyl or mono-, di-, tri- or tetra-substituted 1,1'-binaphthyl-2,2'-diyl of formula V, whereby R^{A} and R^{A'} are independently from each other selected from the group consisting of methyl, phenyl, 4-nitrophenyl, 4-trifluoromethylphenyl, 3,5-bis(trifluoromethyl)phenyl, 9-anthracenyl and 2,4,6-trimethylphenyl, and R^{B} and R^{B'} are independently from each other selected from the group consisting of bromo, iodo, nitro, phenyl, methoxy and 3,5-bis(trifluoromethyl)phenyl, 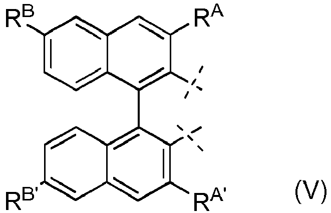
with the proviso that at least one of R⁴ and R⁵ is not hydrogen (see also **Fig. 1**).

"Aryl" is preferably selected from the group consisting of 1-naphthyl and 2-naphthyl.

The present invention is especially directed towards an industrial sustainable process for the manufacture of 6-methyl-5-hepten-2-one ("MH"; see **Fig. 2**), 6-methyl-5-octen-2-one ("EH"; see **Fig. 3**), 6,10-dimethyl-5,9-undecadien-2-one (= geranylacetone ("GA") or "nerylacetone" ("NA"), respectively; see **Fig. 4**), and 6,10,14-trimethyl-5,9,13-pentadeca-trien-2-one (= farnesylacetone; "FA"; see **Fig. 5**).

The preferred catalysts according to the present invention are "1-NDHP" (1-naphthyl dihydrogen phosphate) and "BNHP" ((*RS*)-1,1'-binaphthyl-2,2'-diyl hydrogen phosphate).

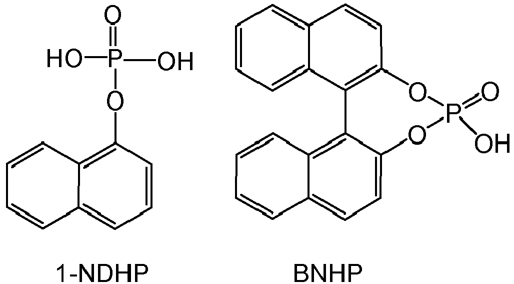

### Detailed description

The present invention is directed towards a process for the manufacture of gamma,delta-unsaturated ketones of the general formula (III) 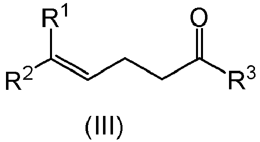 by reacting a tertiary vinyl carbinol of the general formula (I) with an isopropenyl alkyl ether of the general formula (II) in the presence of a catalyst of the general formula (IV), 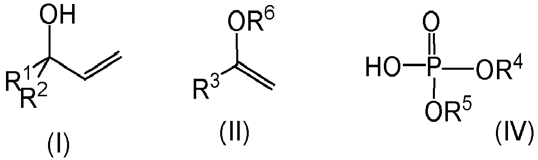
wherein R¹ and R⁶ are independently from each other either methyl or ethyl, R³ is methyl,
R² is selected from the group consisting of methyl, ethyl, homoprenyl, homogeranyl, homoneryl, homofarnesyl, and homosolanesyl,
R⁴ and R⁵ are independently from each other selected from the group consisting of hydrogen and aryl, or R⁴ and R⁵ form together 1,1'-binaphthyl-2,2'-diyl or mono-, di-, tri- or tetra-substituted 1,1'-binaphthyl-2,2'-diyl of formula V, whereby R^{A} and R^{A'} are independently from each other selected from the group consisting of methyl, phenyl, 4-nitrophenyl, 4-trifluoromethylphenyl, 3,5-bis(trifluoromethyl)phenyl, 9-anthracenyl and 2,4,6-trimethylphenyl, and R^{B} and R^{B'} are independently from each other selected from the group consisting of bromo, iodo, nitro, phenyl, methoxy and 3,5-bis(trifluoromethyl)phenyl, 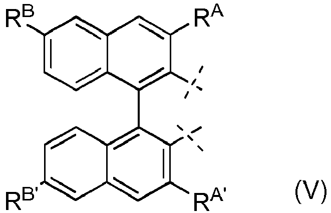
with the proviso that at least one of R⁴ and R⁵ is not hydrogen.

### Startling materials

### Compound of the formula (I)

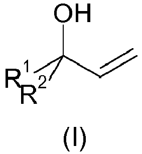

R¹ is either methyl or ethyl, and R² is selected from the group consisting of methyl, ethyl, homoprenyl, homogeranyl, homoneryl, homofarnesyl, and homosolanesyl, as listed in the table 1 below.

**Table 1:**

| **R²** | |
|---|---|
| methyl | CH₃ |
| ethyl | CH₂CH₃ |
| CH₂-prenyl = "homoprenyl" | 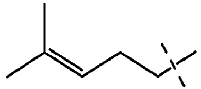 |
| CH₂-geranyl/CH 2-neryl = "homogeranyl"/" homoneryl" | 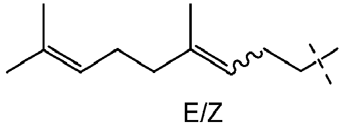 |
| CH₂-farnesyl = "homofarnesyl" | 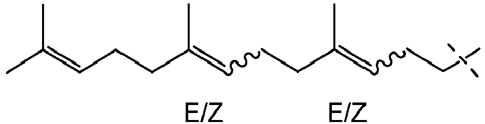 |
| CH₂-solanesyl = "homosolanesyl" | 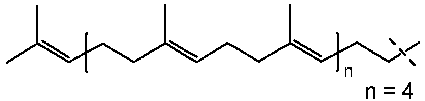 |

Preferably R¹ is methyl and R² is either methyl, ethyl, homoprenyl, or homogeranyl.

Thus, the compound of the general formula (I) is preferably selected from the group consisting of 2-methyl-3-buten-2-ol ("MBE"), 3-methyl-4-penten-3-ol ("EBE"), 3,7-dimethyl-1,6-octadien-3-ol (= linalool, "LL"), and (6*E*/6*Z*)-3,7,11-trimethyl-1,6,10-dodecatrien-3-ol (= *E*/*Z*-nerolidol, "*E*/*Z*-NL").

More preferably, the compound of formula (I) is MBE or EBE, even more preferred is MBE.

### Compound of the formula (II)

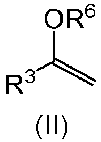

The compound of formula (II) is preferably either isopropenyl methyl ether ("IPM"; R³ = methyl) or isopropenyl ethyl ether ("IPE"; R³ = ethyl), whereby isopropenyl methyl ether is more preferred.

### Amounts of compounds of the formula (I) and (II)

The molar ratio of the tertiary vinyl carbinol of the general formula (I) to the isopropenyl alkyl ether of the general formula (II) is preferably ranging from 1:7 to 1:1, more preferably ranging from 1:5 to 1:1.5, most preferably ranging from 1:3.5 to 1:2.

### Catalysts of the formula (IV)

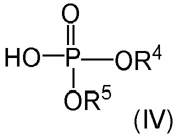

R⁴ and R⁵ are independently from each other selected from the group consisting of hydrogen and aryl, or R⁴ and R⁵ form together 1,1'-binaphthyl-2,2'-diyl or mono-, di-, tri- or tetra-substituted 1,1'-binaphthyl-2,2'-diyl of formula V, whereby R^{A} and R^{A'} are independently from each other selected from the group consisting of methyl, phenyl, 4-nitrophenyl, 4-trifluoromethylphenyl, 3,5-bis(trifluoromethyl)phenyl, 9-anthracenyl and 2,4,6-trimethylphenyl, and R^{B} and R^{B'} are independently from each other selected from the group consisting of bromo, iodo, nitro, phenyl, methoxy and 3,5-bis(trifluoromethyl)phenyl, 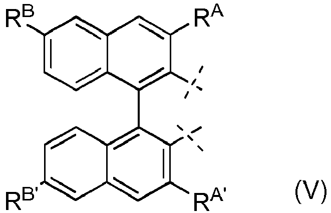 with the proviso that at least one of R⁴ and R⁵ is not hydrogen
"Aryl" is preferably selected from the group consisting of 1-naphthyl and 2-naphthyl.

The preferred catalysts according to the present invention are "1-NDHP" (1-naphthyl dihydrogen phosphate) and "BNHP" ((*RS*)-1,1'-binaphthyl-2,2'-diyl hydrogen phosphate).

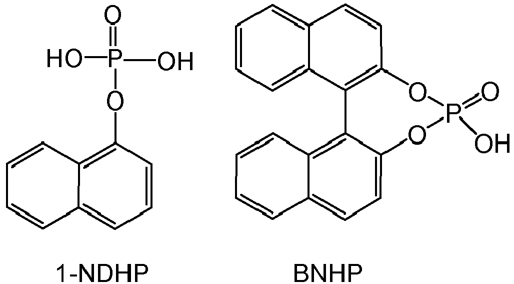

The amount of the catalyst of formula (IV) is preferably ranging from 0.01 - 0.3 mol-%, more preferably ranging from 0.01-0.1 mol-%, most preferably ranging from 0.02-0.07 mol-%, based on the amount of the tertiary vinyl carbinol of the general formula (I).

Advantageously the catalysts of formula (IV) are used as such, i.e. in solid form, and not as solution in an organic solvent such as aliphatic ketones as e.g. acetone.

### Reaction conditions

### Temperature

The reaction is preferably carried out at a temperature ranging from 100 to 170°C, more preferably at a temperature ranging from 110 to 160°C, most preferably at a temperature ranging from 120 to 150°C.

### Pressure

The reaction is preferably carried out at a pressure ranging from 5 to 15 bar, more preferably at a pressure ranging from 8 to 12 bar.

### Solvent

The reaction can be carried out without solvent or in the presence of an organic solvent. Preferably the reaction is carried out without solvent.

Even if the reaction is carried out in the absence of an organic solvent, the starting materials, the compounds of formula (I) and (II), as well as the catalyst of formula (IV) may still be provided in an organic solvent. Thus, in the reaction mixture there may be an amount of organic solvent present up to 10 weight-%, preferably up to 5 weight-%, more preferably up to 3 weight-%, based on the total weight of the reaction mixture.

If the reaction is carried out in an organic solvent, polar aprotic organic solvents such as aliphatic ketones as e.g. acetone are preferred.

The reaction mixture itself, with or without an organic solvent, is also an object of the present invention. Thus, the present invention is directed towards a reaction mixture comprising a compound of formula (I), a compound of formula (II) and a catalyst of formula (IV), as well as towards a reaction mixture consisting essentially of a compound of formula (I), a compound of formula (II) and a catalyst of formula (IV) 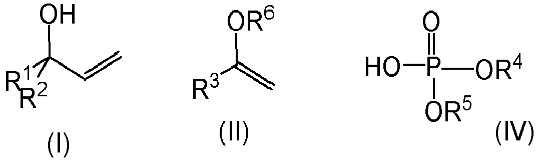 "consisting essentially of" in this context means that the total amount of the compound of formula (I), the compound of formula (II) and the catalyst of formula (IV) sum up to an amount of at least 90 weight-%, preferably at least 95 weight-%, more preferably at least 97 weight-%, based on the total amount of the reaction mixture.

The present invention is also directed towards the use of a catalyst of the formula (IV) 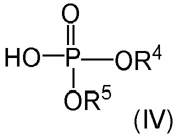 wherein R⁴ and R⁵ are independently from each other selected from the group consisting of hydrogen and aryl, or R⁴ and R⁵ form together 1,1'-binaphthyl-2,2'-diyl or mono-, di-, tri- or tetra-substituted 1,1'-binaphthyl-2,2'-diyl of formula V, whereby R^{A} and R^{A'} are independently from each other selected from the group consisting of methyl, phenyl, 4-nitrophenyl, 4-trifluoromethylphenyl, 3,5-bis(trifluoromethyl)phenyl, 9-anthracenyl and 2,4,6-trimethylphenyl, and R^{B} and R^{B'} are independently from each other selected from the group consisting of bromo, iodo, nitro, phenyl, methoxy and 3,5-bis(trifluoromethyl)phenyl, 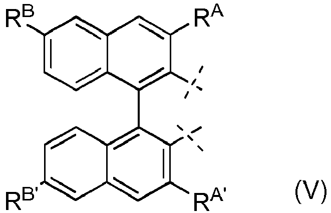 with the proviso that at least one of R⁴ and R⁵ is not hydrogen,
for the manufacture of gamma,delta-unsaturated ketones of the general formula (III) 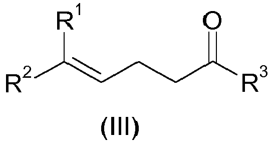 by reacting a tertiary vinyl carbinol of the general formula (I) with an isopropenyl alkyl ether of the general formula (II) 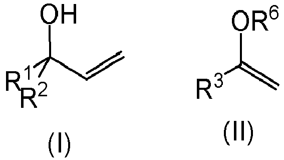 wherein R¹ and R⁶ are independently from each other either methyl or ethyl, R³ is methyl, and
R² is selected from the group consisting of methyl, ethyl, homoprenyl, homogeranyl, homoneryl, homofarnesyl, and homosolanesyl, whereby the preferences as given above also apply.

The present invention is now further illustrated by the following nonlimiting examples.

### Examples:

### General procedure

The powdery catalyst (either 1-NDHP or BNHP in the amounts as given in the tables below) is filled into the reactor as such. 144 g of the starting material (either MBE, EBE, LL or E-NL as given in the tables below) and the amount of IPM ("eq." refers to the molar ratio relative to the other starting material, the compound of formula (I)) as given in the tables below are mixed and poured into the reactor. The closed reactor is then evacuated and released with nitrogen three times. The reaction mixture is stirred and heated up to the temperature as given in the tables below. The experiment is then carried out at isothermal conditions. After the reaction time given in the tables, the reaction mixture is cooled down to 20°C. A sample of the reaction mixture is collected and directly neutralized with sodium acetate. The sample is then analyzed by gas chromatography.

**Table 2: Conditions: 2.1 eq. IPM, catalyst = 1-NDHP, starting material: MBE, product: MH**

| **Example** | **1** | **2** |
|---|---|---|
| Amount of catalyst [mol-%] | 0.03 | 0.06 |
| Temperature [°C] | 150 | 120 |
| Conversion MBE [%] | 96.99 | 91.14 |
| Yield MH [%] | 94.80 | 89.90 |
| Selectivity | 0.977 | 0.986 |

**Table 3: Conditions: 2.1 eq. IPM; catalyst = 1-NDHP, starting material: EBE, product: EH**

| **Example** | **3** |
|---|---|
| Amount of catalyst [mol-%] | 0.03 |
| Temperature [°C] | 150 |
| Conversion EBE [%] | 92.9 |
| Yield EH [%] | 92.0 |
| Selectivity | 0.99 |

**Table 4: Conditions: 3.8 eq. IPM, catalyst = 1-NDHP, starting material = LL, product = GA**

| **Example** | **4** |
|---|---|
| Amount of catalyst [mol-%] | 0.1 |
| Temperature [°C] | 160 |
| Conversion LL [%] | 96.7 |
| Yield GA [%] | 91.6 |
| Selectivity | 0.95 |

**Table 5: Conditions: 3.8 eq. IPM, catalyst = 1-NDHP, starting material = E-NL, product = FA**

| **Example** | **5** |
|---|---|
| Amount of catalyst [mol-%] | 0.1 |
| Temperature [°C] | 160 |
| Conversion E-NL [%] | 99.2 |
| Yield FA [%] | 93.4 |
| Selectivity | 0.94 |

**Table 6: Conditions: 2.1 eq. IPM, catalyst = BNHP, starting material: MBE, product: MH**

| **Example** | **6** | **7** | **8** |
|---|---|---|---|
| Amount of catalyst [mol-%] | 0.03 | 0.03 | 0.01 |
| Temperature [°C] | 120 | 120 | 120 |
| Conversion MBE [%] | 98.86 | 97.50 | 95.83 |
| Yield MH [%] | 89.43 | 89.81 | 91.67 |
| Selectivity | 0.905 | 0.921 | 0.957 |

**Table 7: Conditions: 2.1 eq. IPM, catalyst = BNHP, starting material = EBE, product = EH**

| **Example** | **9** |
|---|---|
| Amount of catalyst [mol-%] | 0.03 |
| Temperature [°C] | 120 |
| Conversion EBE [%] | 97.7 |
| Yield EH [%] | 93.0 |
| Selectivity | 0.95 |

**Table 8: Catalyst = BNHP, starting material = LL, product = GA**

| **Example** | **10** | **11** | **12** |
|---|---|---|---|
| Amount of IPM [eq.] | 2.5 | 2.5 | 3.8 |
| Amount of catalyst [mol-%] | 0.05 | 0.1 | 0.1 |
| Temperature [°C] | 160 | 160 | 160 |
| Conversion LL [%] | 94.2 | 99.5 | 99.2 |
| Yield GA [%] | 89.1 | 85.2 | 92.7 |
| Selectivity | 0.95 | 0.86 | 0.93 |

**Table 9: Catalyst = BNHP, starting material = E-NL, product = FA**

| **Example** | **13** |
|---|---|
| Amount of IPM [eq.] | 3.8 |
| Amount of catalyst [mol-%] | 0.1 |
| Temperature [°C] | 160 |
| Conversion E-NL [%] | 99.1 |
| Yield FA [%] | 92.5 |
| Selectivity | 0.93 |

## Claims

1. A process for the manufacture of gamma,delta-unsaturated ketones of the general formula (III) 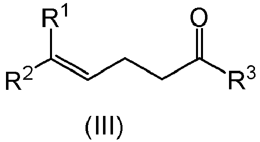 by reacting a tertiary vinyl carbinol of the general formula (I) with an isopropenyl alkyl ether of the general formula (II) in the presence of a catalyst of the general formula (IV), 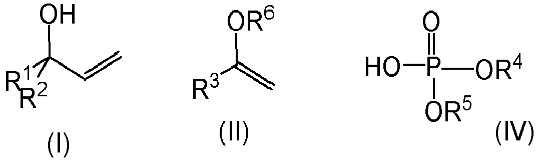
wherein R¹ and R⁶ are independently from each other either methyl or ethyl, R³ is methyl,
R² is selected from the group consisting of methyl, ethyl, homoprenyl, homogeranyl, homoneryl, homofarnesyl, and homosolanesyl, and
R⁴ and R⁵ are independently from each other selected from the group consisting of hydrogen and aryl, or R⁴ and R⁵ form together 1,1'-binaphthyl-2,2'-diyl or mono-, di-, tri- or tetra-substituted 1,1'-binaphthyl-2,2'-diyl of formula V, whereby R^{A} and R^{A'} are independently from each other selected from the group consisting of methyl, phenyl, 4-nitrophenyl, 4-trifluoromethylphenyl, 3,5-bis(trifluoromethyl)phenyl, 9-anthracenyl and 2,4,6-trimethylphenyl, and R^{B} and R^{B'} are independently from each other selected from the group consisting of bromo, iodo, nitro, phenyl, methoxy and 3,5-bis(trifluoromethyl)phenyl, 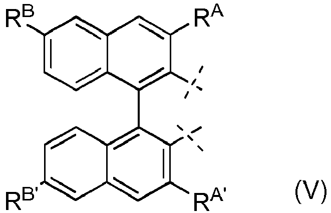
with the proviso that at least one of R⁴ and R⁵ is not hydrogen.

2. The process according to claim 1, wherein the catalyst of the formula (IV) is either 1-naphthyl dihydrogen phosphate or 1,1'-binaphthyl-2,2'-diyl hydrogen phosphate.

3. The process according to claim 1 and/or 2, wherein R¹ is methyl and R² is selected from the group consisting of methyl, ethyl, homoprenyl, and homogeranyl.

4. The process according to any one or more of the preceding claims, wherein the reaction is carried out at a temperature ranging from 100 to 170°C, preferably at a temperature ranging from 110 to 160°C, more preferably at a temperature ranging from 120 to 150°C.

5. The process according to any one or more of the preceding claims, wherein the reaction is carried out at a pressure ranging from 5 to 15 bar, preferably at a pressure ranging from 8 to 12 bar.

6. The process according to any one or more of the preceding claims, wherein the molar ratio of the tertiary vinyl carbinol of the general formula (I) to the isopropenyl alkyl ether of the general formula (II) is ranging from 1:7 to 1:1, preferably ranging from 1:5 to 1:1.5, more preferably ranging from 1:3.5 to 1:2.

7. The process according to any one or more of the preceding claims, wherein the amount of the catalyst is ranging from 0.01-0.3 mol-%, preferred ranging from 0.01-0.1 mol-%, more preferred ranging from 0.02-0.07 mol-%, based on the amount of the tertiary vinyl carbinol of the general formula (I).

8. The process according to any one or more of the preceding claims, wherein the compound of the general formula (III) is selected from the group consisting of 6-methyl-5-hepten-2-one, 6-methyl-5-octen-2-one, 6,10-dimethyl-5,9-undecadien-2-one and 6,10,14-trimethyl-5,9,13-pentadeca-trien-2-one, preferably wherein the compound of the general formula (III) is 6-methyl-5-hepten-2-one or 6-methyl-5-octen-2-one, more preferably wherein the compound of the general formula (III) is 6-methyl-5-hepten-2-one.

9. Use of a catalyst of the formula (IV) 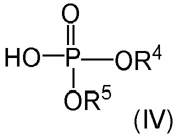 wherein R⁴ and R⁵ are independently from each other selected from the group consisting of hydrogen and aryl, or R⁴ and R⁵ form together 1,1'-binaphthyl-2,2'-diyl or mono-, di-, tri- or tetra-substituted 1,1'-binaphthyl-2,2'-diyl of formula V, whereby R^{A} and R^{A'} are independently from each other selected from the group consisting of methyl, phenyl, 4-nitrophenyl, 4-trifluoromethylphenyl, 3,5-bis(trifluoromethyl)phenyl, 9-anthracenyl and 2,4,6-trimethylphenyl, and R^{B} and R^{B'} are independently from each other selected from the group consisting of bromo, iodo, nitro, phenyl, methoxy and 3,5-bis(trifluoromethyl)phenyl, 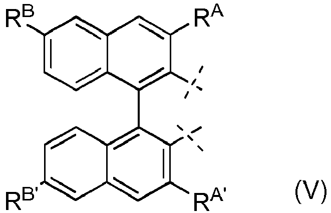 with the proviso that at least one of R⁴ and R⁵ is not hydrogen,
for the manufacture of gamma,delta-unsaturated ketones of the general formula (III) 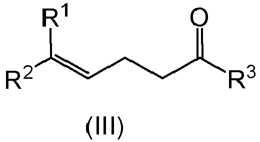 by reacting a tertiary vinyl carbinol of the general formula (I) with an isopropenyl alkyl ether of the general formula (II) 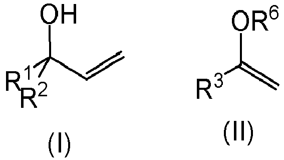 wherein R¹ and R⁶ are independently from each other either methyl or ethyl, R³ is methyl, and R² is selected from the group consisting of methyl, ethyl, homoprenyl, homogeranyl, homoneryl, homofarnesyl, and homosolanesyl.

10. A reaction mixture comprising a compound of formula (I), a compound of formula (II) and a catalyst of formula (IV) 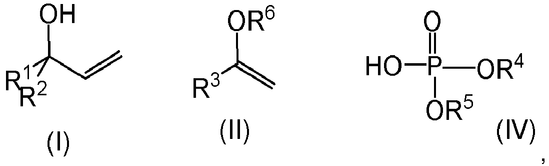
wherein R¹ and R⁶ are independently from each other either methyl or ethyl, R³ is methyl, and
R² is selected from the group consisting of methyl, ethyl, homoprenyl, homogeranyl, homoneryl, homofarnesyl, and homosolanesyl, and
R⁴ and R⁵ are independently from each other selected from the group consisting of hydrogen and aryl, or R⁴ and R⁵ form together 1,1'-binaphthyl-2,2'-diyl or mono-, di-, tri- or tetra-substituted 1,1'-binaphthyl-2,2'-diyl of formula V, whereby R^{A} and R^{A'} are independently from each other selected from the group consisting of methyl, phenyl, 4-nitrophenyl, 4-trifluoromethylphenyl, 3,5-bis(trifluoromethyl)phenyl, 9-anthracenyl and 2,4,6-trimethylphenyl, and R^{B} and R^{B'} are independently from each other selected from the group consisting of bromo, iodo, nitro, phenyl, methoxy and 3,5-bis(trifluoromethyl)phenyl, 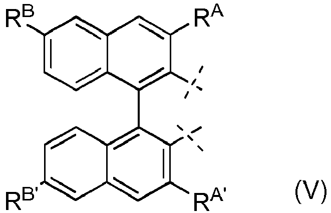
with the proviso that at least one of R⁴ and R⁵ is not hydrogen.

11. A reaction mixture consisting essentially of a compound of formula (I), a compound of formula (II) and a catalyst of formula (IV) 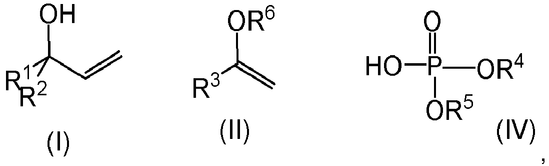
wherein R¹ and R⁶ are independently from each other either methyl or ethyl, R³ is methyl, and R² is selected from the group consisting of methyl, ethyl, homoprenyl, homogeranyl, homoneryl, homofarnesyl, and homosolanesyl, and
R⁴ and R⁵ are independently from each other selected from the group consisting of hydrogen and aryl, or R⁴ and R⁵ form together 1,1'-binaphthyl-2,2'-diyl or mono-, di-, tri- or tetra-substituted 1,1'-binaphthyl-2,2'-diyl of formula V, whereby R^{A} and R^{A'} are independently from each other selected from the group consisting of methyl, phenyl, 4-nitrophenyl, 4-trifluoromethylphenyl, 3,5-bis(trifluoromethyl)phenyl, 9-anthracenyl and 2,4,6-trimethylphenyl, and R^{B} and R^{B'} are independently from each other selected from the group consisting of bromo, iodo, nitro, phenyl, methoxy and 3,5-bis(trifluoromethyl)phenyl, 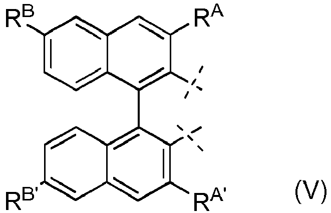
with the proviso that at least one of R⁴ and R⁵ is not hydrogen.
